# EUROPÄISCHE PATENTSCHRIFT

(11) **EP 3 675 767 B1**
(45) Veröffentlichungstag und Bekanntmachung des Hinweises auf die Patenterteilung: **19.07.2023**
(21) Anmeldenummer: 18829797.2
(22) Anmeldetag: 17.12.2018
(51) Int. Cl.: A61C 5/62, A61M 5/315, A61M 5/31

(54) **NACHLAUFFREIE SPRITZE, INSBESONDERE ZUR VERABREICHUNG DENTALER PASTÖSER ZUSAMMENSETZUNGEN, UND KIT**
OVER-TRAVEL-FREE SYRINGE, IN PARTICULAR FOR ADMINISTERING PASTE-LIKE DENTAL COMPOSITIONS AND KIT
SERINGUE HERMÉTIQUE, EN PARTICULIER POUR L'ADMINISTRATION DE COMPOSITIONS PÂTEUSES DENTAIRES ET KIT

(30) Priorität: 21.12.2017 DE 102017130944
(43) Veröffentlichungstag der Anmeldung: 08.07.2020
(73) Patentinhaber: Kulzer GmbH, 63450 Hanau (DE)
(72) Erfinder: KEMENY, Andrea, 61381 Friedrichsdorf (DE); KONDZIELA, Mike, 35781 Weilburg (DE); UTTERODT, Andreas, 61267 Neu-Anspach (DE); ZUMKELLER, Hans Joachim, 61276 Weilrod (DE); FORTH, Heiko, 61273 Wehrheim (DE); LAUTENSCHLÄGER, Kai, 63755 Alzenau (DE); KUPKA, Jörg, 60435 Frankfurt (DE)
(74) Vertreter: Bendele, Tanja
(86) Internationale Anmeldenummer: PCT/EP2018/085274
(87) Internationale Veröffentlichungsnummer: WO 2019/121550

(56) Entgegenhaltungen:
- EP-A2- 2 329 791

## Beschreibung

Spritze mit Spritzenkörper und einem proximalen Spritzenauslass im Spritzenkörper sowie mit einem axial im Spritzenkörper beweglichen Spritzenstempel mit einem proximalen Ende und einem distalen Ende, wobei innerhalb des Spritzenstempels ausgehend vom proximalen Ende des Spritzenstempels bis zum distalen Ende des Spritzenkolbens ein längliches Lumen ausgebildet ist, in dem ein beweglicher innerer Stempel mit einem proximalen Ende und einem distalen Ende angeordnet ist.

Im medizinischen Alltag gibt es mehrfach verwendbare Spritzen, die in der Regel mit einem Präparat, wie einer dentalen Zusammensetzung, bspw. einem Fissurenversiegler, einem Füllungsmaterial oder einem Wirkstoff, wie einem Anästhetikum, gefüllt sind. Die Applikation von pastösen oder flüssigen Zubereitungen, wie eines Fissurenversieglers, erfolgt häufig über mehrfach verwendbare Spritzen mit Hilfe von Kanülen. Die Kanülen werden jeweils gewechselt. Die Kanülen werden entweder auf den Spritzenauslass aufgesteckt oder über einen Luer-Lock Verschluss, der den Spritzenauslass bildet, sicher verankert.

Die Viskositäten der Zusammensetzungen können stark variieren von sehr festen, stopfbaren Kompositen bis hin zu sogenannten Flow-Materialien, diese weisen eine fließfähige Konsistenz auf. Fließfähige dentale Zusammensetzungen werden in der Regel in vorgefüllten Spritzen in den Verkehr gebracht und können üblicherweise mehrfach angewendet werden. Eine Spritze enthält daher eine Menge, die für eine Vielzahl von Anwendungen ausreichend ist, so dass nur die Kanülen an der Spritze ausgewechselt werden müssen.

Beim Herauspressen der Dentalmasse baut sich im Spritzenkörper, synonym zu Spritzenzylinder, ein hoher Innendruck auf. Nachdem der Anwender eine ausreichende Menge der Dentalmasse appliziert hat, wird nachfolgend durch den Innendruck in der Spritze weitere Dentalmasse aus der Spritze ausgefördert, obwohl vom Anwender kein Druck mehr auf die Spritze ausgeübt wird. Bei handelsüblichen Spritzen wird zur Verbesserung des Gleitverhaltens zwischen Spritzenkörper und Spritzenkolben bzw. Spritzenstempel der Spritzenkörper silikonisiert. Durch diese Maßnahme kann der Spritzenkolben weiter aus dem Spritzenköper durch den Innendruck herausbefördert werden, um den Innendruck selbsttätig abzubauen. Die Silikonisierung ist aber nicht in allen Anwendungsgebieten möglich, da diese die Produkteigenschaften der zu applizierenden Dentalmasse erheblich beeinträchtigen kann. Beispielsweise kann die Haftung der ausgehärteten Dentalmasse im Zahn stark durch diese Gleitmittel oder Antihaftmittel beeinträchtig werden. Daher kann diese bekannte Silikonisierung der inneren Oberfläche des Spritzenkörpers die Produkteigenschaften der Dentalmasse stark verschlechtern. Damit ist durch die fehlende Silikonisierung des Spritzenkörpers die Reibung zwischen der inneren Oberfläche des Spritzenkörpers und des Spritzenstempels sehr hoch. Die hohe Reibung verhindert dann nach der Benutzung ein selbsttätiges Zurückschieben des Spritzenstempels bzw. Spritzenkolbens und somit die Einstellung eines Druckausgleichs. Der verbleibende hohe Innendruck im Spritzenkörper fördert dann die Dentalmasse aus dem Spritzenkörper durch die Kanüle in den Außenbereich. Dieses Verhalten wird als Nachlaufen bezeichnet und ist vom Anwender unerwünscht.

Der Wettbewerber Voco beschreibt in seinem Patent EP2016962B1 eine technische Lösung mit einem Reibschlusselement mit Federelement für dieses Problem. EP2329791 A2 offenbart eine Spritze mit den Merkmalen des Oberbegriffs des Anspruch 1, die ein Nachtropfen von Dentalmaterial aus der Spritze verhindern soll.

Grundsätzlich können auch in anderen Anwendungsbereichen fließfähige und/oder pastöse Zusammensetzungen aus einer Spritze abgegeben werden, wie beim Löten, Aufbringen von pastösen Zusammensetzungen, wie beispielsweise Wärmeleitpasten, Klebstoffen oder Abdichtungsmassen oder Fugenmassen, die als 1K- oder 2K-Komponenten vorliegen können.

Aufgabe der Erfindung war es, eine weitere Spritze bereitzustellen, die vorgenannte Nachlaufproblematiken vermeidet und vorzugsweise eine mehrfache Verwendung von Spritzen mit Zusammensetzungen ohne Nachlaufen der Zusammensetzung aus der Spritze ermöglicht. Die Nachlaufproblematik kann zu einem Verkleben der Zusammensetzung mit der Unterlage und ggf. zu einer ungewollten Kontamination der Zusammensetzung führen. Zudem wird unnötig Zusammensetzung ausgetragen, die am Spritzenauslass oder einer angebrachten Kanüle wieder entfernt werden muss bevor mit der Spritze weitergearbeitet werden kann.

Gelöst wurde die Aufgabe mit einer Nachlauf-freien Spritze nach Anspruch 1 sowie einem Kit nach Anspruch 14. Bevorzugte Ausführungsformen der Erfindung sind in den abhängigen Ansprüchen definiert.

Unter einer Nachlauf-freien Spritze wird eine Spritze verstanden aus der nach dem Applizieren einer flüssigen oder pastösen Zusammensetzung, insbesondere einer pharmazeutischen oder dentalen Zusammensetzung, ohne Anwendung einer von außen angewendeten Kraft auf den Spritzenstempel und den inneren beweglichen Stempel die Zusammensetzung nicht aus dem Spritzenauslass austritt. Während der Applikation der Zusammensetzung wird der Spritzenstempel und der innere bewegliche Stempel von außen mit einer Kraft beaufschlagt, insbesondere vom Anwender mit einer Kraft beaufschlagt.

Gegenstand der Erfindung ist eine Spritze nach Anspruch 1.

Nach einer Ausführungsform wird eine Spritze beansprucht, bei der die Reibung (A) zwischen dem Spritzenstempel und dem Spritzenkörper größer ist als die Reibung (B) zwischen dem inneren Stempel im Lumen des Spritzenstempels, wobei die Reibung (B) ein Verschieben des inneren Stempels nach distal ermöglicht, insbesondere wenn keine nach proximal gerichtete äußere Kraft zum Einbringen des inneren Stempels in den Spritzenkörper anliegt, ohne, dass der Spritzenstempel im Spritzenkörper nach distal verschoben wird. Der innere Stempel wirkt als Innendruckentlastungselement.

Beansprucht wird somit eine Spritze mit folgender Funktionsweise:
- Beaufschlagen des Spritzenstempels und des inneren Stempels mit einer Kraft zum Einbringen des Spritzenstempels und des inneren Stempels in den Spritzenkörper, um definiert eine im Spritzenkörper bevorratete Zusammensetzung aus dem Spritzenauslass oder einer damit verbundenen Kanüle auszutragen,
- Beenden der Beaufschlagung des Spritzenstempels und des inneren Stempels mit einer Kraft, wobei der im Spritzenkörper aufgebaute Innendruck, insbesondere der Zusammensetzung, den inneren Stempel im Lumen des Spritzenstempels axial nach distal bewegt und der Innendruck im Spritzenkörper abgebaut wird, insbesondere um ein Nachlaufen der Zusammensetzung aus dem Spritzenauslass oder der Kanüle zumindest teilweise zu vermeiden.

Die Nachlauf-Problematik wird mit der erfindungsgemäßen Spritze umfassend einen hülsenförmigen Spritzenkörper und einen hohlen Spritzenstempel vermieden, indem sich im Innern des Spritzenstempels bzw. des Kolbens in der Kolbenaufnahme ein axial bewegliches Element befindet. Dieses Element reicht bis zur Griffplatte des Kolbens und wird beim Betätigen der Spritze zusammen mit dem Kolben in der Kolbenaufnahme nach proximal geschoben. Nach der Anwendung schiebt der Innendruck im Spritzenkörper das bewegliche Element nach distal, wodurch der Innendruck reduziert wird. Dazu ist die Reibung zwischen dem beweglichen Element und dem Kolben mit Kolbenaufnahme geringer eingestellt als die Reibung zwischen dem Spritzenauslass, insbesondere durch die Kanüle, und der Dentalmasse. Ein Nachlaufen der Dentalmasse durch den Spritzenauslass, insbesondere durch die Kanüle nach außen wird somit verhindert. Beim erneuten Betätigen der Spritze wird vom Anwender erst das bewegliche Element, insbesondere der innere Stempel, nach proximal geschoben bis diese bündig mit dem Kolben oder der Kolbenaufnahme abschließt. Da die Gleitflächen zwischen dem beweglichen Element und dem Kolben mit Kolbenaufnahme nicht mit der Dentalmasse in Berührung kommen, kann zur Reduzierung der Reibung auch ein Gleitmittel, wie Silikonöl in diesem Bereich eingesetzt werden.

Gegenstand der Erfindung ist eine Spritze mit Spritzenkörper, insbesondere ist der Spritzenkörper in Form einer Hülse ausgebildet, der Spritzenkörper weist einem proximalen Spritzenauslass sowie einen axial im Spritzenkörper beweglichen Spritzenstempel mit einem proximalen Ende und einem distalen Ende auf, innerhalb des Spritzenstempels ausgehend vom proximalen Ende des Spritzenstempels bis zum distalen Ende des Spritzenkolbens ist ein längliches Lumen ausgebildet, in dem ein beweglicher innerer Stempel mit einem proximalen Ende und einem distalen Ende angeordnet ist. Vorzugsweise weist der Spritzenkörper proximal einen Spritzenauslass auf, der als Konnektor zum Festlegen einer Kanüle mit Konnektor oder einer Applikationsleitung oder Entnahmeleitung jeweils mit Konnektor ausgebildet ist.

Dabei ist es bevorzugt, wenn im Spritzenkörper, der insbesondere in Form einer Hülse vorliegt, der axial im Spritzenkörper beweglichen Spritzenstempel zumindest in einem Bereich, insbesondere in einem Bereich bis vollständig, mit seiner äußeren Oberfläche umlaufend kraftschlüssig an der inneren Oberfläche des Spritzenkörpers anliegt. Vorzugsweise liegt der proximale Stempel des Spritzenstempels und optional die mindestens eine Dichtlippe umlaufend kraftschlüssig an der inneren Oberfläche des Spritzenkörpers an. Dabei ist es bevorzugt, wenn die Dichtlippe außen am äußeren Umfang umlaufend am Spritzenstempel angeordnet, insbesondere angeformt, besonders bevorzugt angeklebt oder angeschweißt ist. Des Weiteren ist es bevorzugt, wenn innerhalb des Spritzenstempels ausgehend vom proximalen Ende des Spritzenstempels bis zum distalen Ende des Spritzenstempels/-kolbens ein längliches Lumen ausgebildet ist, in dem ein beweglicher innerer Stempel mit einem proximalen Ende und einem distalen Ende angeordnet ist. Das innerhalb des Spritzenstempels vorliegende vom proximalen Ende bis zum distalen Ende des Spritzenstempels verlaufende längliche Lumen ist vorzugsweise in mindestens einem bis mehreren Abschnitten zylindrisch ausgebildet. Besonders bevorzugt ist das innerhalb des Spritzenstempels vorliegende vom proximalen Ende bis zum distalen Ende des Spritzenstempels verlaufende längliche Lumen durchgängig zylindrisch ausgebildet. Unter zylindrisch wird erfindungsgemäß ein allgemeiner senkrechter Zylinder verstanden, d.h. alle Mantellinien stehen senkrecht auf einem polygonen Querschnitt, wobei ein Prisma gebildet wird. Besonders bevorzugt liegt das Lumen vom proximalen bis zum distalen Ende als ein senkrechter Kreiszylinder vor. Vorzugsweise ist die innere Oberfläche des Lumens eben, insbesondere glatt. Vorzugsweise behindert weder ein Vorsprung und/oder eine andere Stufe eine Bewegung des inneren Stempels nach distal. Nach einer Ausführungsform ist es bevorzugt, wenn proximal an der Stirnseite in Richtung des Spritzenauslass es weder am Spritzenstempel noch am inneren Stempel ein Dichtelement vorliegt.

Die äußere Oberfläche dieses inneren Stempels liegt zumindest teilweise bis vollständig und/oder eine umlaufende Dichtlippe auf der äußeren Oberfläche des inneren Stempels kraftschlüssig an der inneren Oberfläche des Lumens des Spritzenstempels an.

Der Kraftschluss zwischen der äußeren Oberfläche des inneren Stempels und der inneren Oberfläche des Lumens kleiner als der Kraftschluss zwischen der inneren Oberfläche des Spritzenkörpers und der äußeren Oberfläche des Spritzenstempels.

Nach einer besonders bevorzugten Ausführungsform entspricht bei der erfindungsgemäßen Spritze die Betätigungskraft des inneren Spritzenstempels, insbesondere im länglichen Lumen des Spritzenkolbens, kleiner gleich 60% der Betätigungskraft des Spritzenstempels, insbesondere im Spritzenkörper. Besonders bevorzugt beträgt die aufzuwendende Betätigungskraft des inneren Spritzenstempels 1 bis 60 % der aufzuwendenden Betätigungskraft des Spritzenstempels, bevorzugt 20 bis 60 %, besonders bevorzugt 30 bis 60 %. Die Betätigungskraft kann auch der Auspresskraft aus der Spritze entsprechen. Gemessen werden kann die Bestätigungskraft mit einer Zwick-Zug- und Druckprüfmaschine mit der die aufzuwendende Kraft gemessen wird, die benötigt wird, um a) den Stempel oder Kolben im Spritzenkörper nach vorne zu schieben (Betätigungskraft des Spritzenstempels) oder den inneren Stemple im Lumen des Spritzenkolbens nach proximal oder distal, insbesondere nach distal zu schieben (Betätigungskraft des inneren Spritzenstempels), wobei der Stempel selbst fixiert ist.

Ferner ist es bevorzugt, wenn der innere Stempel proximal an seinem äußeren Umfang keine integral am inneren Stempel ausgebildeten Vorsprünge aufweist, insbesondere kann der innere Stempel aber eine am äußeren Umfang angeformte umlaufende Dichtlippe aufweisen. Das Material der Dichtlippe kann bevorzugt Silicon umfassen.

Des Weiteren ist es bevorzugt, wenn der innere Stempel und der Spritzenstempel eine identische Länge aufweisen oder der innere Stempel kürzer bis gleichlang wie der Spritzenstempel ist. Besonders bevorzugt liegen das proximale Ende des inneren Stempels und der proximale Stempel des Spritzenstempels bzw. der proximale Kolben des Spritzenkolbens in der Lager- und/oder Handhabungsposition in einer gemeinsamen Ebene, insbesondere bilden sie eine gemeinsame Ebene an ihrer Stirnseite.

Nach der Applikation der Zusammensetzung in der Nachlauf-freien Position der Spritze kann das proximale Ende, insbesondere die Stirnseite, des inneren Stempels nach distal in das Lumen des Spritzenstempels hineingeschoben vorliegen. In der Nachlauf-freien Position kann daher die Ebene des proximalen Endes, insbesondere der Stirnseite, des inneren Stempels gegenüber der Ebene, insbesondere der Stirnseite, des proximalen Stempels des Spritzenstempels bzw. der Ebene des proximalen Kolbens des Spritzenkolbens nach distal verschoben sein.

Der innere Stempel ist zumindest teilweise bis vollständig außen auf seiner äußeren Oberfläche und/oder zumindest teilweise bis vollständig die innere Oberfläche des Lumens mit einer Gleitkomponente umfassend ein Gleitmittel oder einem in der Matrix vorliegenden Additiv oder Mischung von Additiven und/oder oberflächlich mit einem Additiv oder einer Mischung von Additiven versehen, um die Reibung zwischen der äußeren Oberfläche des inneren Stempels und der inneren Oberfläche des Lumens zu vermindern.

Nach einer erfindungsgemäßen Alternative ist zumindest teilweise bis vollständig der innere Stempel außen auf seiner äußeren Oberfläche und/oder zumindest teilweise bis vollständig die innere Oberfläche des Lumens mit einem Gleitmittel versehen, um die Reibung zwischen der äußeren Oberfläche des inneren Stempels und der inneren Oberfläche des Lumens zu vermindern.

Nach einer Ausführungsform ragt der innere Stempel nicht bis in den proximalen Spritzenauslass hinein und/oder der bewegliche Spritzenstempel ragt nicht bis in den proximalen Spritzenauslass hinein.

Nach einer bevorzugten Ausführungsform weist der innere Stempel am distalen Ende einen Flügel auf. Der Spritzenstempel kann auch als Kolben mit proximaler Kolbenaufnahme ausgebildet sein.

Nach einer Ausführungsform ist innerhalb des Spritzenstempels ausgehend vom proximalen Ende des proximalen Stempels des Spritzenstempels bis durch den Flügel am distalen Ende des Spritzenkolbens ein längliches Lumen ausgebildet, in dem ein beweglicher innerer Stempel mit einem proximalen Ende und einem distalen Ende mit Flügel angeordnet ist.

Der Spritzenkörper mit eingesetztem axial beweglichen Spritzenstempel begrenzt vorzugsweise einen Spritzenkörperinnenraum mit einem Volumen zur Aufnahme einer flüssigen bis pastösen Zusammensetzung, insbesondere mit einem Volumen von 0,01 mL bis 2 L, vorzugsweise von 0,01 mL bis 500 mL, bevorzugt von 0,01 bis 10 mL, besonders bevorzugt mit einem Volumen von 0,01 mL bis 2 mL. Besonders bevorzugt weist der Spritzenköper mit eingesetztem, axial beweglichen Spritzenstempel ein Volumen von 0,01 mL bis 2 mL auf, insbesondere beträgt das Volumen 0,2 mL bis 1,5 mL, bevorzugt sind 0,5 bis 1,2 mL, besonders bevorzugt um 1 mL. Der Spritzenköper liegt vorzugsweise in Form einer Hülse vor, bevorzugt in Form einer zylindrischen einseitig mit einem Spritzenauslass versehenen Hülse. Der innere Durchmesser des Spritzenkörpers im Bereich, in dem sich der axial bewegliche Spritzenstempel befindet, beträgt vorzugsweise 4 bis 14 mm, bevorzugt 5 bis 8 mm. Der Außendurchmesser der Kanüle beträgt vorzugsweise 0,4 bis 1,4 mm (27G bis 17G), bevorzugt 0,6 bis 1,2 mm (23G bis 18G), besonders bevorzugt 0,9 mm (20G). G ist das Gaugenmaß und entspricht der Anzahl der Ziehstufen vom Rohrrohling. Die Kanüle ist vorzugsweise aus einem Metall gefertigt.

Ferner ist es bevorzugt, wenn das längliche Lumen axial, insbesondere axial entlang der gesamten Längsachse des Spritzenstempels, im Spritzenstempel vom proximalen bis zum distalen Ende ausgebildet ist. Vorzugsweise ist der Spritzenstempel rotationssymmetrisch, bevorzugt umfasst er mindestens einen rotationssymmetrischen Spritzenkolben. Des Weiteren ist das längliche Lumen vorzugsweise rotationssymmetrisch ausgebildet. Als Spritzenkolben gilt der Spritzenstempel ohne distale Flügel, die der Spritzenstempel zusätzlich aufweisen kann. Spritzenstempel und Spritzenkolben können sich daher entsprechen, insbesondere sofern die distalen Flügel optional am Spritzenstempel vorgesehen sind. Ein erfindungsgemäßer Spritzenkolben kann vorzugsweise kreiszylindrisch mit erfindungsgemäßem Lumen ausgebildet sein. Somit ist innerhalb des Spritzenstempels ausgehend vom proximalen Ende des Spritzenstempels bis zum distalen Ende des Spritzenkolbens bzw. des Spritzenstempels ein längliches Lumen ausgebildet, da insbesondere der Spritzenkolben dem Spritzenstempel ohne Flügel entspricht und Teil des Spritzenstempels mit Flügeln ist.

Zudem kann der Spritzenstempel a) am proximalen Ende mindestens einen Stempel und optional mindestens eine umlaufende Dichtlippe im proximalen Bereich des Spritzenstempels und am distalen Ende einen Flügel aufweisen, oder b) der Spritzenstempel kann in Form mindestens seines Kolbens vorliegen, der optional am proximalen Ende einen Stempel und optional mindestens eine umlaufende Dichtlippe im proximalen Bereich des Spritzenstempels und optional am distalen Ende einen Flügel aufweisen.

In weiteren Ausgestaltungen kann der Spritzenstempel a) am proximalen Ende mindestens einen Stempel und optional mindestens eine am äußeren Umfang umlaufende Dichtlippe oder einen am äußeren Umfang angeordneten O-Ring und am distalen Ende einen Flügel aufweisen, oder b) der Spritzenstempel kann in Form mindestens eines Kolbens vorliegen, der optional am proximalen Ende einen Stempel und optional mindestens eine am äußeren Umfang umlaufende Dichtlippe oder am äußeren Umfang angeordneten O-Ring und optional am distalen Ende einen Flügel aufweist. Vorzugsweise weist der innere Stempel zumindest teilweise, insbesondere teilweise bis vollständig, außen auf seiner äußeren Oberfläche und/oder zumindest teilweise, insbesondere teilweise bis vollständig, oder die Dichtlippe und/oder der O-Ring, ein Gleitmittel auf, und/oder die innere Oberfläche des Lumens weist zumindest teilweise ein Gleitmittel auf. Das Gleitmittel kann eine fluide Substanz oder eine fluide Zusammensetzung sein, wie ein Silikonöl. Das Gleitmittel vermindert die Reibung zwischen der äußeren Oberfläche des inneren Stempels und der inneren Oberfläche des Lumens. Das Gleitmittel gilt als ein Additiv.

Ferner ist es bevorzugt, wenn innerhalb des Spritzenstempels ausgehend vom proximalen Ende des Spritzenkolbens, insbesondere von der proximalen Oberfläche, bis zum distalen Ende des Spritzenkolbens, insbesondere bis zur distalen Oberfläche des Spritzenkolbens, mindestens ein durchgängiges längliches Lumen durch den gesamten Spritzenstempel verläuft, und wobei in dem Lumen ein beweglicher innerer Stempel mit einem proximalen Ende und einem distalen Ende, insbesondere mit Flügel am distalen Ende, angeordnet ist. Vorzugsweise sind das Lumen und der innere Stempel axial im Spritzenstempel angeordnet, insbesondere sind sie gegeneinander beweglich angeordnet. Insbesondere verläuft das Lumen durch den Stempel des Spritzenstempels bis durch den Flügel, bevorzugt ist das Lumen axial im Spritzenstempel angeordnet und in dem länglichen axialen Lumen ein beweglicher innerer Stempel mit einem proximalen Ende und einem distalen Ende, insbesondere mit Flügel am distalen Ende, angeordnet, wobei der innere Stempel axial beweglich ist. Bevorzugt sind der Spritzenstempel und der innere Stempel gleich lang, so dass insbesondere das proximale und das distale Ende des Spritzenstempels und des inneren Stempels in der gleichen Ebene enden. Bevorzugt weist der Spritzenstempel nur ein längliches Lumen zur Aufnahme eines inneren Stempels auf.

Bevorzugt ist der Kraftschluss zwischen der äußeren Oberfläche des inneren Stempels und der inneren Oberfläche des Lumens kleiner als der Kraftschluss zwischen der inneren Oberfläche des Spritzenkörpers und der äußeren Oberfläche des Spritzenstempels. Der Spritzenauslass am Spritzenkörper kann als ein Konnektor oder ein Teil eines Konnektorsystems ausgebildet sein. Als Konnektor oder Konnektorsystem kommen grundsätzlich die folgenden in Betracht: Steckverbindung, Rastung, Drehverbindung, Dreh-/ Rastverbindung, jegliche Luer-Systeme, wie ein Luer-Lock-System, ein rotierendes Luer-Lock-System, Stecksystem umfassend zwei Hohlkegel, Schraubverschluss oder ein Stecksystem mit Rastnocke, eine Bajonettverbindung. Ein Konnektor kann innen als weiblicher Luer-Lock-Anschluss und außen als männlicher Luer-Lock-Anschluss ausgebildet sein. Der weibliche Luer-Lock-Anschluss kann an den männlichen Luer-Lock-Anschluss des Spritzenauslasses des Spritzenkörpers festgelegt werden. Ein weiterer Konnektor, bspw. einer Kanüle, kann als weiblicher Luer-Lock-Anschluss ausgebildet sein, um diesem mit dem männlichen Luer-Lock-Anschluss des vorstehenden Konnektors zu verbinden. Die Spritze oder der Spritzenauslass, insbesondere in Form eines Konnektors können jeweils unabhängig eine Schutzkappe vorzugsweise mit Konnektor aufweisen. Eine Spritze kann mit einem Konnektor, vorzugsweise aus Kunststoff, und mit einer Kanüle, insbesondere aus Metall, ausgestattet sein. Daher kann auch das Kit einen solchen Konnektor mit Kanüle enthalten.

Unter verbindbar wird im Rahmen der Erfindung eine Verbindung zweier vorgenannter Teile verstanden, die dicht gegenüber einem Austritt von Flüssigkeit verbunden sind und deren Verbindung wieder lösbar ist.

Nach einer besonders bevorzugten Ausführungsform ist am proximalen Spritzenauslass, am Konnektor oder am Teil eines Konnektorsystems eine Kanüle optional mit einem Konnektor oder mit einem an das Teil des Konnektorsystems passendes Teil angeordnet. Der proximale Spritzenauslass liegt vorzugsweise als Konnektor oder Teil eines Konnektorsystems vor.

Ferner ist es bevorzugt, wenn die Reibung (A) zwischen dem Spritzenstempel und dem Spritzenkörper größer ist als die Reibung (B) des inneren Stempels im Lumen des Spritzenstempels, insbesondere ist die Reibung (A) 10% größer ist als die Reibung (B), vorzugsweise ist die Reibung (A) 50% größer als die Reibung (B).

Nach einer alternativen Ausführungsformen könne jeweils unabhängig der Spritzenkörper, der Spritzenstempel und/oder der innere Stempel aus einem thermoplastischen Kunststoff, wie TPE (thermoplastische Elastomere), gefertigt sein. Bevorzugte thermoplastische Kunststoffe umfassen: PE (Polyethylen), PP (Polypropylen), Polyacetale, insbesondere POM (Polyoxymethylen), PBT (Polybutylenterephthalat), PET (Polyethylenterephthalat), PETP (Polyethylenterephthalat), PA (Polyamid) und/oder PTFE (Polytetrafluorethylen). Gleichfalls können die Kunststoffe in der Matrix oder oberflächlich mit mindestens einem Additiv oder einer Mischung von Additiven versehen sein, vorzugsweise sogenannte Gleitmittel, zur Reduzierung der Reibung, wie beispielsweise: Ölsäureamid(e)/Oelamid(e) Erucamid ((Z)-docos-13-enamid), Ethylene-bis-oleamid und/oder Stearyl-Erucamid ((Z)-N-octadecyldocos-13-enamid), Festkörperschmierstoffe, wie Polytetrafluorethylen (PTFE), Molybändisulfid (MoS), Bornitrid (BN), Siliconöl oder auch weitere ölige bis wachsartige Substanzen, die in die Polymermatrix eincompoundiert werden können. PTFE wir nur als Schmiermittel in den Kunststoff in der Matrix eincompoundiert, wenn die Matrix nicht bereits aus diesem Kunststoff besteht. Alternativ kann PFTE als oberflächliche Kunststoffschicht auf einen Kern aus einem anderen thermoplastischen Kunststoff aufgebracht werden. Bevorzugt kann die Spritze, der Spritzenkörper, Spritzenstempel/-kolben und/oder der innere Stempel, vorzugsweise der Spritzenkörper, der Spritzenstempel und/oder der innere Stempel, jeweils unabhängig aus einem Kunstoffen gefertigt sein, der einen sehr geringen Reibungskoeffizienten aufweist, besonders bevorzugt sind POM, PBT, PET, PETP, PA, PTFE und/oder PE und/oder PP oder Mischungen oder Co-Polymere von mindesten zwei der Kunststoffe. Besonders bevorzugt können der innere Stempel und/oder der Spritzenstempel/-kolben jeweils unabhängig aus einem Kunstoffen gefertigt sein, der in der Matrix oder oberflächlich mit mindestens einem Additiv oder einer Mischung von Additiven versehen sein kann, vorzugsweise sind das bzw. die Additive sogenannte Gleitmittel. Vorzugsweise sind das proximale Ende des Spritzenstempels/-kolbens und/oder das proximale Ende des inneren Stempels oberflächlich nicht mit Additiven versehen. Gleichfalls kann der Spritzenkörper vorzugsweise das Additiv nicht aufweisen.

Optional können die beiden proximalen Enden des Spritzenstempels/-kolbens und des inneren Stempels mit einer zusätzlichen Folie ohne Additive versehen sein, damit vorzugsweise die flüssige bis pastöse Zusammensetzung nicht mit Additiven in Kontakt kommt. Sofern das mindestens eine Additiv in der Matrix vorliegt, kann es in einer Alternative bevorzugt sein, wenn das Additiv aus der Matrix zusätzlich an die Oberfläche migriert. Ein besonders bevorzugter Kunststoff für den Spritzenkörper, Stempel und/oder inneren Stempel kann eine zweiphasig aufgebaute Kunststoff-Komponente sein, die vorzugsweise oberflächlich eine verminderte Reibung aufweist und im Kern aus einem Kunststoff mit gegenüber dem oberflächlich verwendeten Kunststoff erhöhten mechanischen Eigenschaften aufweist. Beispiele für entsprechend aufgebaute Spritzenkörper und/oder Spritzenstempel und/oder innere Stempel sind Kunststoff-Komponenten mit einem Polyacetal-Kern, wie POM, mit einer optional mit anorganischen Partikeln gefüllten PTFE- Randschicht.

Nach weiteren alternativen Ausführungsformen kann a) das distale Ende des Spritzenstempels einen Flügel aus einem thermoplastischen Kunststoff aufweisen, und/oder b) das proximale Ende des Spritzenstempels proximal und/oder im proximalen Bereich am äußeren Umfang umlaufend eine Dichtlippe oder einen O-Ring aufweisen, insbesondere ist die Dichtlippe oder der O-Ring aus einem thermoplastischen und/oder elastischem Kunststoff. Bevorzugte Kunststoffe umfassen einen thermoplastischen Kunststoff, ein Elastomer oder thermoplastisches Elastomer. Bevorzugt kann die Dichtlippe oder der O-Ring aus einem Silicon gebildet sein. Dabei ist es bevorzugt, wenn die Dichtlippe ein integrales Teil des Spritzenstempels ist. Der O-Ring kann als separates Bauteil, vorzugsweise in eine umlaufende Nut an den Spritzenstempel angebracht werden.

Um den inneren Stempel während der Lagerung oder unterstützend beim Austragen einer Zusammensetzung aus der Spritze am Spritzenstempel, insbesondere bis zu einer definierten Kraft, zu fixieren, kann a) der Flügel des inneren Stempels am distalen Ende eine seitliche Rastnocke aufweisen, die innen am distalen Ende im Lumen im Flügel des Spritzenstempels, insbesondere im Lagerzustand der Spritze, in eine Vertiefung eingerastet ist, oder b) am distalen Ende ist innen im Lumen des Flügels des Spritzenstempels eine seitliche Rastnocke vorgesehen, die außen am distalen Ende in eine Vertiefung im Flügel des inneren Stempels, insbesondere im Lagerzustand der Spritze, eingerastet ist oder einrasten kann, oder c) der Spritzenstempel kann als mindestens ein Kolben ausgebildet sein, der innen im Lumen am distalen Ende mindestens eine seitliche Rastnocke aufweist, die auf der Außenseite des inneren Stempels am distalen Ende in eine Vertiefung, insbesondere im Lagerzustand eingerastet ist oder einrasten kann, oder d) der innere Stempel kann als mindestens ein Kolben ausgebildet sein, der auf der Außenseite mindestens eine Rastnocke aufweist, die innen im Lumen in mindestens eine Vertiefung im Spritzenstempel, insbesondere im Lagerzustand, eingerastet ist oder einrasten kann. Die Rastnocke und die korrespondierende Vertiefung oder die korrespondierenden Vertiefungen können am Spritzenstempel und inneren Stempel an beliebiger Position angeordnet sein.

Gleichfalls Gegenstand der Erfindung ist ein Kit umfassend eine Spritze, wobei die Spritze gefüllt ist mit einer flüssigen oder pastösen pharmazeutischen oder dentalen Zusammensetzung, wobei die dentale Zusammensetzung mindesten ein polymerisierbares Monomer umfasst. Vorzugsweise ist die Spritze gefüllt mit einem dentalen Fissurenversiegler, einer Gingiva-Retraktionspaste, Adstringenz, dentalen Kleber, selbstätzenden Adhäsiv, Gewebekleber, insbesondere Cyanacrylat-Hautkleber, Kleberproteinlösung, Thrombinlösung mit Kalciumchlorid, pharmazeutischer Wirkstoff, wie ein Anästhetikum oder Antibiotika.

Die flüssigen oder pastösen pharmazeutischen oder dentalen Zusammensetzungen weisen vorzugsweise eine Viskosität im Bereich von 12700 bis 65000 mPas auf, vorzugsweise im Bereich von 12700 bis 40000 mPas. Uneingefärbte Pasten können eine Viskosität von bis zu 60000 mPas aufweisen. Die Viskosität kann in Anlehnung an die DIN EN ISO 3219_1994-10, DIN 53019-1_2008-09 oder DIN 1342-1_2003-11 bestimmt werden. Die Prüfung kann vorzugsweise mit folgenden Prüfparametern erfolgen: Belastungsviskosität: Scherrate d(Gamma)dt = 0,1 ...... 50 1/s linear, Zeitvorgabe = 30 Messpunkte, Messpunktdauer = 6 Sekunden. Die Messung kann weiterhin vorzugsweise bei 20 bis 23 °C, bevorzugt bei 23 °C und vorzugsweise mit einem Rheometer wie Anton Paar - Physica MCR 301 erfolgen.

Der erfindungsgemäße Aufbau der Spritze erlaubt einerseits dem Anwender, dass er erkennt, wenn die Spritze sich nach der Anwendung entspannt hat, indem das distale Ende des beweglichen inneren Stempels am distalen Ende des Spritzenstempels hervorsteht. Ein besonderer weiterer Vorteil des inneren Stempels ist zudem, dass der Anwender über die erneute Beaufschlagung nur des herausstehenden distalen Endes des inneren Stempels eine Feindosierung bei der Applikation vornehmen kann. Der Spritzenstempel ist vorzugsweise axial entlang der Längsachse des Spritzenkolbens und/oder des inneren Stempels beweglich.

Die Auspresskraft einer Zusammensetzung aus der Spritze bei gebogener/abgewinkelter Kanüle, insbesondere mit einem Winkel im Bereich von 30 bis 54°, und einem Außendurchmesser von 0,9 mm (20G) kann vorzugsweise 30 bis 150 N betragen.

Unter einer nachlauffreien Applikation wird verstanden, dass weniger als 0,5 mg, vorzugsweise weniger als 0,4 mg je Austragevorgang aus der Spritze austreten.

Die nachfolgenden Figuren erläutern die Erfindung näher, ohne die Erfindung auf die Ausführungsbeispiele zu beschränken.

Die Figuren stellen dar:
- Figur 1a, 1b:: Spritzen **1** mit Spritzenkörper **5** und Spritzenkolben **7** mit Spritzenauslass **11** in Form eines Konnektors **4** gemäß dem Stand der Technik
- Figur 2a, 2b, 2c:: Erfindungsgemäße Spritze **1** mit Spritzenauslass **11** in Form eines Konnektors **4** oder Konnektorsystems **4a** mit Spritzenstempel **7** mit Lumen **14** und innerem Stempel **8**
- Figur 3:: Spritze **1** mit Kanüle **2a** und Verschluss **10**

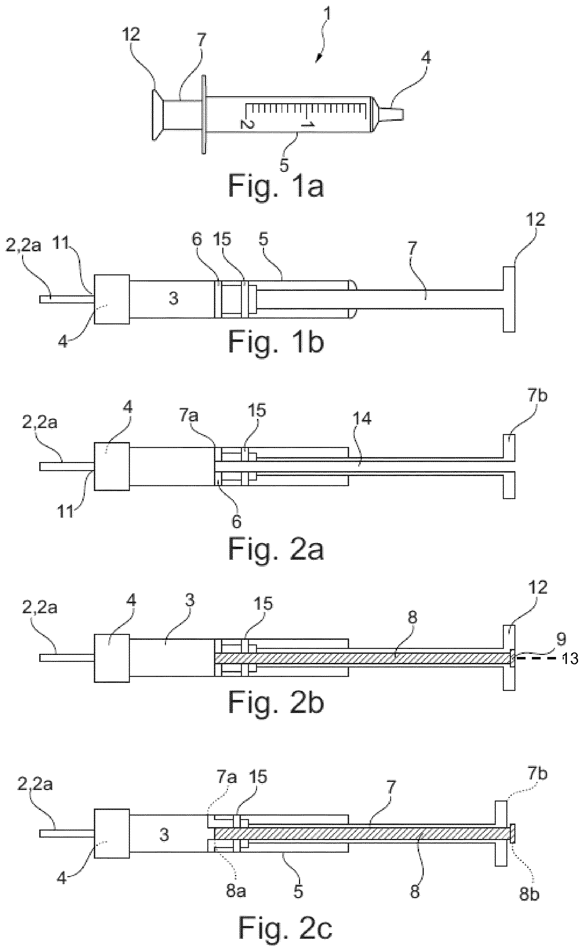

In den **Figuren 1a** und **1b** werden Spritzen **1** nach dem Stand der Technik offenbart. Die Spritzen **1** weisen einen Spritzenkörper **5** mit Spritzenstempel **7** oder Kolben mit Kolbenaufnahme **7** mit distalem Flügel **12** auf. An der Spritze in **Figuren 1a** und **2a** ist der Konnektor **4** als Luer-Lock-Konnektor dargestellt. In Figur **1b** ist der Spritzenkörper **5** als Hülse mit Spritzenauslass **11** als Konnektor **4** mit Aufsatz **2** in Form einer Kanüle **2a** dargestellt. Im Spritzenkörper **5** ist die Zusammensetzung **3** eingefüllt. Der proximale Stempel **6** ggf. mit Dichtlippe **15** bzw. die proximale Kolbenaufnahme **6** dient zusammen mit dem Spritzenstempel **7** und dem distalen Flügel **12** dem Herauspressen der Zusammensetzung **3.** Da die Zusammensetzung 3 durch eine Kanüle mit sehr geringerem Durchmesser ausgetragen werden soll, wird im Innern des Spritzenkörpers **5** ein hoher Innendruck aufgebaut, der nach dem Applizieren und zu Nachlaufen der Zusammensetzung aus der Kanüle führt, obwohl auf den Spritzenstempel **7** keine Kraft mehr ausgeübt wird.

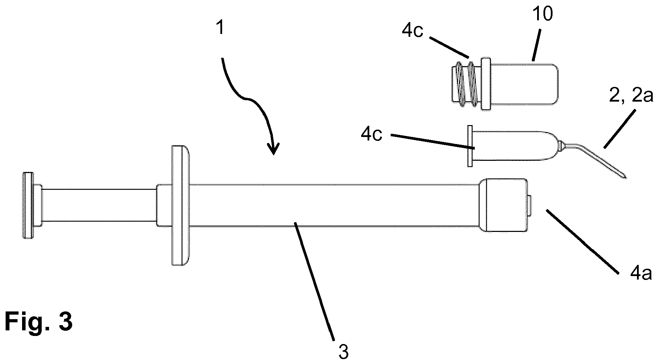

In den **Figuren 2a, 2b** und **2c** werden erfindungsgemäße Spritzen **1** mit einem Spritzenkörper **5** und einem Spritzenauslass **11** in der Geometrie eines Konnektors **4** mit aufgesetztem Aufsatz **2,** hier eine Kanüle **2a** mit eigenem Konnektor dargestellt. Im Spritzenkörper **5** ist eine pastöse Zusammensetzung **3** bevorratet, die mithilfe des Spritzenkolbens **7** aus der Kanüle **2a** mit sehr geringem Innendurchmesser ausgetragen werden soll. Der Spritzenstempel **7** kann im proximalen Bereich einen proximalen Stempel **6** am proximalen Ende **7a** sowie eine Dichtlippe **15** im proximalen Bereich aufweisen. Der Anwender übt dabei gemeinsam auf die beiden Flügel **9** und **12** am distalen Ende **8b** des inneren Stempels **8** und distalen Ende **7b** des Spritzenstempels **7** eine Kraft aus, die ausreichend ist eine Menge der Zusammensetzung zur Applikation aus der Kanüle auszutragen. Nach der Applikation kann der Anwender die Spritze **1** ablegen. Durch den aufgebauten Innendruck im Spritzenkörper **5** kann der innere Stempel **8** axial innerhalb des Spritzenstempels **7** nach, insbesondere entlang der Längslachse **13** des Spritzenstempels und des inneren Stempels, distal bewegt werden. Auf diese Weise wird der Druck abgebaut und die Zusammensetzung fließt nicht mehr aus der Kanüle aus, sondern kann sich innerhalb des Spritzenkörpers entlasten. In dieser Position liegt das proximale Ende **7a** des Spritzenstempels **7** vor dem proximalen Ende **8a** des inneren Stempels **8,** wie in **Figur 2c** dargestellt. Der Anwender kann bei Bedarf nun das distale Ende **8b** des inneren Stempels **8** für eine Feinapplikation wieder in den Spritzenstempel **7** einschieben. **Figur 3** stellt dar eine Spritze **1** mit Spritzenkörper **3** und einem Konnektor 4a zur Aufnahme eines Verschlusses **10** mit Konnektor **4c** sowie eines Aufsatzes **2** mit Kanüle **2a** und Konnektor **4c.**

### Bezugszeichen

- **1**: Spritze
- **2**: Aufsatz, insbesondere Kanüle **2a**
- **3**: Zusammensetzung in Spritzenkörper
- **4**: Konnektor, oder Teil eines Konnektorsystems **4a,** Konnektor **4b, 4c** Teil - passend zu Teil **4a** des Konnektorsystems, Konnektor am proximalen Spritzenkörper mit Innen- oder Außengewinde am proximalen Ende
- **5**: Spritzenkörper
- **6**: proximaler Stempel des Spritzenstempels/Kolben des Spritzenkolbens
- **7**: Spritzenkolben/Spritzenstempel, Kolben mit Kolbenaufnahme, **7a** proximales Ende des Spritzenstempels/-kolbens, **7b** distales Ende des Spritzenstempels/-kolbens,
- **8**: innerer Stempel, insbesondere mit distalem Flügel, **8a** proximales Ende des inneren Stempels, **8b** distales Ende des Stempels
- **9**: distaler Flügel des inneren Stempels
- **10**: Verschluss
- **11**: Spritzenauslass
- **12**: distaler Flügel des Spritzenkolbens/Spritzenstempels
- **13**: Längsachse
- **14**: längliches Lumen
- **15**: Dichtlippe, O-Ring

## Patentansprüche

1. Spritze (1) mit Spritzenkörper (5) und einem proximalen Spritzenauslass (11) im Spritzenkörper (5) sowie mit einem axial (13) im Spritzenkörper beweglichen Spritzenstempel (7) mit einem proximalen Ende und einem distalen Ende,
wobei innerhalb des Spritzenstempels (7) ausgehend vom proximalen Ende (7a) des Spritzenstempels (7) bis zum distalen Ende (7b) des Spritzenkolbens ein längliches Lumen (14) ausgebildet ist, in dem ein beweglicher innerer Stempel (8) mit einem proximalen Ende (8a) und einem distalen Ende (8b) angeordnet ist, **dadurch gekennzeichnet, dass** zumindest teilweise der innere Stempel (8) außen auf seiner äußeren Oberfläche und/oder zumindest teilweise die innere Oberfläche des Lumens (14) mit einer Gleitkomponente umfassend ein Gleitmittel oder einem in der Matrix vorliegenden Additiv oder Mischung von Additiven und/oder oberflächlich mit einem Additiv oder einer Mischung von Additiven versehen sind, um die Reibung zwischen der äußeren Oberfläche des inneren Stempels und der inneren Oberfläche des Lumens zu vermindern, sodass der Kraftschluss zwischen der äußeren Oberfläche des inneren Stempels (8) und der inneren Oberfläche des Lumens (14) kleiner ist als der Kraftschluss zwischen der inneren Oberfläche des Spritzenkörpers (5) und der äußeren Oberfläche des Spritzenstempels (7).

2. Spritze (1) nach Anspruch 1, **dadurch gekennzeichnet, dass**
das längliche Lumen (14) axial (13) im Spritzenstempel (7) vom proximalen (7a) bis zum distalen Ende (7b) im rotationsymmetrischen Spritzenkolben ausgebildet ist, wobei das längliche Lumen rotationssymmetrisch ausgebildet ist.

3. Spritze (1) nach Anspruch 1 oder 2, **dadurch gekennzeichnet, dass** der proximale Spritzenauslass (11) im Spritzenkörper (5) ein Konnektor (4) oder ein Teil eines Konnektorsystems (4a) ist.

4. Spritze (1) nach einem der Ansprüche 1 bis 3, **dadurch gekennzeichnet, dass**
a) der Spritzenstempel (7) am proximalen Ende mindestens einen Stempel (6) und mindestens eine am äußeren Umfang umlaufende Dichtlippe (15) oder einen am äußeren Umfang angeordneten O-Ring im proximalen Bereich des Spritzenstempels (7) und am distalen Ende einen Flügel (12) aufweist, oder
b) der Spritzenstempel (7) in Form mindestens eines Kolbens vorliegt der am proximalen Ende einen Stempel (6) und mindestens eine am äußeren Umfang umlaufende Dichtlippe (15) oder einen am äußeren Umfang angeordneten O-Ring im proximalen Bereich des Spritzenstempels (7).

5. Spritze (1) nach einem der Ansprüche 1 bis 4, **dadurch gekennzeichnet, dass**
i) zumindest teilweise der innere Stempel (8) außen auf seiner äußeren Oberfläche die Dichtlippe (15) und/oder der O-Ring (15) aufweist und/oder die Dichtlippe (15) und/oder der O-Ring (15) mit einem Gleitmittel versehen sind, und/oder
ii) mindestens eine der folgenden Komponenten umfassend den inneren Stempel (8), Dichtlippe (15), O-Ring (15) und/oder den Spritzenstempel (7) jeweils unabhängig aus mindestens einem Kunstoff gefertigt sind, der in der Matrix mit mindestens einem Additiv oder einer Mischung von Additiven versehen ist, und/oder
iii) mindestens eine der folgenden Komponenten umfassend den inneren Stempel (8), Dichtlippe (15), O-Ring (15) und/oder den Spritzenstempel (7) jeweils unabhängig oberflächlich mit mindestens einem Additiv oder einer Mischung von Additiven versehen sind,
um die Reibung zwischen der äußeren Oberfläche des inneren Stempels (8) und der inneren Oberfläche des Lumens (14) zu vermindern.

6. Spritze (1) nach einem der Ansprüche 1 bis 5, **dadurch gekennzeichnet, dass**
i) jeweils unabhängig der Spritzenkörper (5), der Spritzenstempel (7), die Dichtlippe (15), der O-Ring (15) und/oder der innere Stempel (8) aus mindestens einem thermoplastischen Kunststoff gefertigt sind, die thermoplastische Elastomere, Polyethylen, Polypropylen, Polyacetal, wie POM; PBT, PET, PETP, PA, und/oder PTFE umfassen, und/oder
ii) jeweils unabhängig der Spritzenstempel und/oder der innere Stempel aus mindestens einem thermoplastischen Kunststoff gefertigt sind, die thermoplastische Elastomere, Polyethylen, Polypropylen, Polyacetal, wie POM; PBT, PET, PETP, PA und/oder PTFE umfassen und der Kunststoff in der Matrix mindestens ein Additiv oder eine Mischung von Additiven enthält, und/oder
ii) jeweils unabhängig der Spritzenstempel und/oder der innere Stempel aus mindestens einem thermoplastischen Kunststoff gefertigt sind, die thermoplastische Elastomere, Polyethylen, Polypropylen, Polyacetal, wie POM; PBT, PET, PETP, PA und/oder PTFE umfassen und der Kunststoff oberflächlich mit mindestens einem Additiv oder einer Mischung von Additiven versehen ist.

7. Spritze (1) nach Anspruch 6, **dadurch gekennzeichnet, dass** das Additiv oder die Mischung von Additiven umfasst mindestens ein Gleitmittel oder eine Mischung von Gleitmitteln zur Reduzierung der Reibung, mindestens ein Ölsäureamid, Erucamid ((Z)-docos-13-enamid), Ethylen-bis-Öleamid und/oder Stearyl-Erucamid ((Z)-N-octadecyl-docos-13-enamid), Festkörperschmierstoffe, wie Polytetrafluorethylen (PFTE), Molybändisulfid (MoS), Bornitrid (BN), Siliconöl oder auch weitere ölige bis wachsartige Substanzen.

8. Spritze (1) nach einem der Ansprüche 1 bis 7, **dadurch gekennzeichnet, dass** innerhalb des Spritzenstempels (7) ausgehend vom proximalen Ende (7a) (Oberfläche) bis zum distalen Ende (7b) (Oberfläche) des Spritzenkolbens mindestens ein durchgängiges längliches Lumen (14) durch den gesamten Spritzenstempel (7) verläuft, in dem ein beweglicher innerer Stempel (8) mit einem proximalen Ende (8a) und einem distalen Ende (8b), mit Flügel (9) am distalen Ende, angeordnet ist.

9. Spritze (1) nach einem der Ansprüche 1 bis 8, **dadurch gekennzeichnet, dass** am proximalen Spritzenauslass (11), am Konnektor (4) oder an dem Teil eines Konnektorsystems (4a) eine Kanüle (2a) mit einem Konnektor (4b) oder mit einem an das Teil (4a) des Konnektorsystems passendes Teil (4c) angeordnet ist.

10. Spritze (1) nach einem der Ansprüche 1 bis 9, **dadurch gekennzeichnet, dass** der Spritzenköper (7) mit eingesetztem axial beweglichen Spritzenstempel (7) ein Volumen von 0,01 mL bis 2 mL aufweist, oder das Volumen 0,2 mL bis 1,5 mL beträgt.

11. Spritze (1) nach einem der Ansprüche 1 bis 10, **dadurch gekennzeichnet, dass** die Reibung (A) zwischen dem Spritzenstempel (7) und dem Spritzenkörper (5) größer ist als die Reibung (B) zwischen dem inneren Stempel (8) im Lumen (14) des Spritzenstempels (7), wobei die Reibung (A) 10% größer ist als die Reibung (B) oder die Reibung (A) 50% größer als die Reibung (B) ist.

12. Spritze (1) nach einem der Ansprüche 1 bis 11, **dadurch gekennzeichnet, dass**
a) das distale Ende (7b) des Spritzenstempels (7) einen Flügel (12) aus einem thermoplastischen Kunststoff aufweist, und/oder
b) das proximale Ende (7a) des Spritzenstempels (7) proximal und/oder im proximalen Bereich am äußeren Umfang umlaufend eine Dichtlippe oder einen O-Ring aufweist, wobei die Dichtlippe oder der O-Ring aus einem thermoplastischen Kunststoff, Elastomer oder thermoplastischen Elastomer ist.

13. Spritze (1) nach einem der Ansprüche 1 bis 12, **dadurch gekennzeichnet, dass**
a) der Flügel (9) des inneren Stempels (8) am distalen Ende (8b) eine seitliche Rastnocke aufweist, die innen am distalen Ende im Lumen (14) im Flügel (12) des Spritzenstempels (7) im Lagerzustand der Spritze in eine Vertiefung eingerastet ist, oder
b) am distalen Ende (7b) innen im Lumen des Flügels (12) des Spritzenstempels (7) ist eine seitliche Rastnocke vorgesehen, die außen am distalen Ende (8b) in eine Vertiefung im Flügel (9) des inneren Stempels (8) im Lagerzustand der Spritze eingerastet ist, oder
c) der Spritzenstempel (7) als mindestens ein Kolben ausgebildet ist, der innen im Lumen (14) am distalen Ende (7b) mindestens eine seitliche Rastnocke aufweist, die auf der Außenseite des inneren Stempels (8) am distalen Ende (8b) in eine Vertiefung im Lagerzustand eingerastet ist, oder
d) der innere Stempel (8) als mindestens ein Kolben ausgebildet ist, der auf der Außenseite mindestens eine Rastnocke aufweist, die innen im Lumen (14) in mindestens eine Vertiefung im Spritzenstempel (7) im Lagerzustand, eingerastet ist.

14. Kit umfassend eine Spritze (1) nach einem der Ansprüche 1 bis 13, wobei die Spritze gefüllt ist mit einer flüssigen oder pastösen pharmazeutischen oder dentalen Zusammensetzung, mit einem dentalen Fissurenversiegler, Gingiva-Retraktionspaste, Adstringenz, einem dentalen Kleber, ein selbstätzendes Adhäsiv, ein Gewebekleber, mit einem Cyanacrylat-Hautkleber, Kleberproteinlösung, Thrombinlösung mit Kalciumchlorid, pharmazeutischer Wirkstoff, wie ein Anästhetikum oder Antibiotika.

## Claims

1. Syringe (1) having a syringe body (5) and a proximal syringe outlet (11) in the syringe body (5) as well as having a syringe plunger (7) being axially (13) movable in the syringe body, having a proximal end and a distal end, an elongated lumen (14) is formed inside the syringe plunger (7) coming from the proximal end (7a) of the syringe plunger (7) until the distal end (7b) of the syringe plunger, in which a movable inner plunger (8) having a proximal end (8a) and a distal end (8b) is arranged,
**characterised in that**,
the inner plunger, at least in part or totally, on its outer surface and/or the inner surface of the lumen (14), at least in part or totally, is provided with at least one lubricant comprising a slip component comprising a lubricant or an additive or mixture of additives being present in the matrix and/or is superficially provided with an additive or a mixture of additive to reduce friction between the outer surface of the inner plunger and the inner surface of the lumen, wherein the force-fit between the outer surface of the inner plunger and inner surface of the lumen is smaller than the force-fit between the inner surface of the syringe body and the outer surface of the syringe plunger.

2. Syringe (1) according to claim 1, **characterised in that**
the elongated lumen (14) is axially (13) formed in the syringe plunger (7) from the proximal (7a) to the distal end (7b), wherein the elongated lumen is formed rotationally symmetrically.

3. Syringe (1) according to claim 1 or 2, **characterised in that** the proximal syringe outlet (11) in the syringe body (5) is a connector (4) or a part of a connector system (4a).

4. Syringe (1) according to any one of the claims 1 to 3, **characterised in that**
a) the syringe plunger (7) has at least one plunger (6) at the proximal end and at least one sealing lip (15) circumferenting at the outer periphery or an O-ring being arranged at the outer periphery in the proximal region of the syringe plunger (7), and a wing (12) at the distal end, or
b) the syringe plunger (7) is present in the form of a piston having, optionally, a plunger (6) at the proximal end and, optionally, at least one sealing lip (15) circumferenting at the outer periphery or an O-ring being arranged at the outer periphery in the proximal region of the syringe plunger (7)..

5. Syringe (1) according to any one of the claims 1 to 4, **characterised in that**
i) the inner plunger (8), at least in part, on its outer surface, the sealing lip (15) and/or the O-ring (15), and/or the sealing lip (15) and/or the O-ring (15) is provided with at least one lubricant, and/or
ii) at least one of the following components comprising the inner plunger (8), sealing lip (15), O-ring (15) and/or the syringe plunger (7), each independently, are made of at least one plastic being provided with at least one additive or a mixture of additives in the matrix, and/or
iii) at least one of the following components comprising the inner plunger (8), sealing lip (15), O-ring (15) and/or the syringe plunger (7), each independently, is superficially provided with at least one additive or a mixture of additives
to reduce friction between the outer surface of the inner plunger (8) and the inner surface of the lumen (14).

6. Syringe (1) according to any one of the claims 1 to 5, **characterised in that**
i) the syringe body (5), the syringe plunger (7), the sealing lip (15), the O-ring (15) and/or the inner plunger (8), each independently, are made of at least one thermoplastic plastic comprising thermoplastic elastomers, polyethylene, polypropylene, polyacetal, such as POM, PBT, PET, PETP, PA and/or PTFE, and/or
ii) the syringe plunger and/or the inner plunger, each independently, are made of at least one thermoplastic plastic comprising thermoplastic elastomers, polyethylene, polypropylene, polyacetal, such as POM, PBT, PET, PETP, PA and/or PTFE, and the plastic comprising at least one additive or a mixture of additives in the matrix, and/or
iii) the syringe plunger and/or the inner plunger, each independently, are made of at least one thermoplastic plastic comprising thermoplastic elastomers, polyethylene, polypropylene, polyacetal, such as POM, PBT, PET, PETP, PA and/or PTFE, and the plastic is superficially provided with at least one additive or a mixture of additives.

7. Syringe (1) according to claim 6, **characterised in that**, the at least one additive or a mixture of additives comprising at least one lubricant or a mixture of lubricants to reduce the friction comprising at least one oleamide, erucamide ((Z)-docos-13-enamide), ethylene bis-oleamide and/or stearyl erucamide ((Z)-N-octadecyldocos-13-enamide), solid lubricants, such as polytetrafluoroethylene (PTFE), molybdenum disulfide (MoS), boron nitride (BN), silicone oil or further oily to waxy substances,

8. Syringe (1) according to any one of the claims 1 to 7, **characterised in that** at least one continuous elongated lumen (14) runs inside the syringe plunger (7) coming from the proximal end (7a) (surface) until the distal end (7b) (surface) of the syringe plunger through the whole syringe plunger (7), in which a movable inner plunger (8) having a proximal end (8a) and a distal end (8b) having a wing (9), is arranged.

9. Syringe (1) according to any one of the claims 1 to 8, **characterised in that** a cannula (2a), optionally having a connector (4b) or having a part (4) matching at the part (4a) of the connector system, is arranged at the proximal syringe outlet (11), at the connector (4) or at the part of a connector system (4a).

10. Syringe (1) according to any one of the claims 1 to 9, **characterised in that** the syringe body (7) with the inserted syringe plunger (7) being axially movable has a volume of 0.01 ml to 2 ml or the volume amounts to 0.2 ml to 1.5 ml.

11. Syringe (1) according to any one of the claims 1 to 10, **characterised in that** the friction (A) between the syringe plunger (7) and the syringe body (5) is greater than the friction (B) of the inner plunger (8) in the lumen (14) of the syringe plunger (7), wherein the friction (A) is 10 % greater than friction (B) or the friction (A) is 50 % greater than friction (B).

12. Syringe (1) according to any one of the claims 1 to 11, **characterised in that**
a) the distal end (7b) of the syringe plunger (7) has a wing (12) made of thermoplastic plastic, and/or
b) the proximal end (7a) of the syringe plunger (7) has, proximally and/or in the proximal region at the outer periphery, a circumferential sealing lip or an O-ring, wherein the sealing lip or the O-ring is made of a thermoplastic plastic, elastomer or a thermoplastic elastomer.

13. Syringe (1) according to any one of the claims 1 to 12, **characterised in that**
a) the wing (9) of the inner plunger (8) has a lateral cam at the distal end (8b), being engaged into a recess in the wing (12) of the syringe plunger (7) inside the distal end in the lumen (14) in the state of storing the syringe, or
b) a lateral cam is provided at the distal end (7b) inside the lumen of the wing (12) of the syringe plunger (7), being engaged into a recess in the wing (9) of the inner plunger (8) outside at the distal end (8b), in particular in the state of storing the syringe, or
c) the syringe plunger (7) is formed as being at least one piston having at least one lateral cam at the distal end (7b) inside the lumen (14), being engaged into a recess at the outside of the inner plunger (8) at the distal end (8b), in particular in the state of storing the syringe, or
d) the inner plunger (8) is formed as at least one piston having at least one cam on the outside, being engaged into a recess in the syringe piston inside the lumen (14) in the state of storing.

14. Kit comprising a syringe (1) according to any one of the claims 1 to 13, wherein the syringe is filled with a fluid or pasty pharmaceutical or dental composition filled with a dental fissure sealant, gingiva retraction past, astringent, a dental glue, a self-etching adhesive, a tissue glue, with a cyanoacrylate skin glue, gluten protein solution, thrombin solution with calcium chloride, pharmaceutical active agent, such as an anaesthetic or antibiotic.

## Revendications

1. Seringue (1) comprenant un corps de seringue (5) et une sortie de seringue proximale (11) dans le corps de seringue (5), ainsi qu'un piston de seringue (7) mobile axialement (13) dans le corps de seringue et ayant une extrémité proximale et une extrémité distale, une lumière oblongue (14) étant formée à l'intérieur du piston de seringue (7) en partant de l'extrémité proximale (7a) du piston de seringue (7) jusqu'à l'extrémité distale (7b) du piston de seringue, lumière dans laquelle est disposé un piston intérieur mobile (8) avec une extrémité proximale (8a) et une extrémité distale (8b), **caractérisée en ce qu'**au moins partiellement le piston intérieur (8) est pourvu, à l'extérieur sur sa surface extérieure et/ou au moins partiellement à la surface intérieure de la lumière (14), d'un composant lubrifiant comprenant un lubrifiant ou un additif ou un mélange d'additifs présents dans la matrice et/ou sont pourvus en surface d'un additif ou d'un mélange d'additifs, afin de réduire le frottement entre la surface extérieure du piston intérieur et la surface intérieure de la lumière, de sorte que l'adhérence entre la surface extérieure du piston intérieur (8) et la surface intérieure de la lumière (14) est inférieure à l'adhérence entre la surface intérieure du corps de seringue (5) et la surface extérieure du piston de seringue (7).

2. Seringue (1) selon la revendication 1, **caractérisée en ce que**
la lumière oblongue (14) est formée axialement (13) dans le piston de seringue en partant de l'extrémité proximale (7a) jusqu'à l'extrémité distale (7b) dans le piston de seringue à symétrie de révolution, la lumière oblongue étant formée avec une symétrie de révolution.

3. Seringue (1) selon la revendication 1 ou 2, **caractérisée en ce que** la sortie proximale de la seringue (11) dans le corps de seringue (5) est un connecteur (4) ou une partie d'un système de connecteurs (4a).

4. Seringue (1) selon l'une des revendications 1 à 3, **caractérisée en ce que**
a) le piston de seringue (7) présente à l'extrémité proximale au moins un piston (6) et au moins une lèvre d'étanchéité (15) sur le pourtour extérieur ou un joint torique disposé sur le pourtour extérieur dans la zone proximale du piston de seringue (7) et à l'extrémité distale une aile (12), ou
b) le piston de seringue (7) se présente sous la forme d'au moins un piston qui comporte, à l'extrémité proximale, un piston (6) et au moins une lèvre d'étanchéité (15) sur le pourtour extérieur ou un joint torique disposé sur le pourtour extérieur dans la zone proximale du piston de seringue (7) .

5. Seringue (1) selon l'une des revendications 1 à 4, **caractérisée en ce que**
i) au moins partiellement, le piston intérieur (8) présente, à l'extérieur sur sa surface extérieure, la lèvre d'étanchéité (15) et/ou le joint torique (15) et/ou la lèvre d'étanchéité (15) et/ou le joint torique (15) sont pourvus d'un lubrifiant et/ou
ii) au moins l'un des composants suivants, comprenant le piston interne (8), la lèvre d'étanchéité (15), le joint torique (15) et/ou le piston de seringue (7), est respectivement indépendamment fabriqué à partir d'au moins une matière plastique qui est pourvue dans la matrice d'au moins un additif ou un mélange d'additifs, et/ou
iii) au moins l'un des composants suivants, comprenant le piston intérieur (8), la lèvre d'étanchéité (15), le joint torique (15) et/ou le piston de seringue (7), est respectivement indépendamment pourvu en surface d'au moins un additif ou un mélange d'additifs, afin de réduire le frottement entre la surface extérieure du piston intérieur (8) et la surface intérieure de la lumière (14).

6. Seringue (1) selon l'une des revendications 1 à 5, **caractérisée en ce que**
i) respectivement indépendamment, le corps de seringue (5), le piston de seringue (7), la lèvre d'étanchéité (15), le joint torique (15) et/ou le piston interne (8) sont fabriqués à partir d'au moins une matière thermoplastique, comprenant des élastomères thermoplastiques, du polyéthylène, du polypropylène, du polyacétal, comme le POM, le PBT, le PET, le PETP, le PA et/ou le PTFE, et/ou
ii) respectivement indépendamment, le piston de seringue et/ou le piston interne sont fabriqués à partir d'au moins une matière thermoplastique, comprenant des élastomères thermoplastiques, du polyéthylène, du polypropylène, du polyacétal, tel que le POM, le PBT, le PET, le PETP, le PA et/ou le PTFE, et la matière plastique contient dans la matrice au moins un additif ou un mélange d'additifs, et/ou
ii) respectivement indépendamment, le piston de seringue et/ou le piston interne sont fabriqués à partir d'au moins une matière thermoplastique, comprenant des élastomères thermoplastiques, du polyéthylène, du polypropylène, du polyacétal, comme le POM, le PBT, le PET, le PETP, le PA et/ou le PTFE, et la matière plastique est pourvue en surface d'au moins un additif ou un mélange d'additifs.

7. Seringue (1) selon l'une des revendications 6, **caractérisée en ce que** l'additif ou le mélange d'additifs comprend au moins un lubrifiant ou un mélange de lubrifiants pour réduire le frottement, au moins un amide d'acide oléique, de l'érucamide (((Z)-docos-13-énamide)), éthylène-bis-oléamide et/ou stéaryl-érucamide (((Z)-N-octadécyldocos-13-énamide), des lubrifiants solides, tels que le polytétrafluoroéthylène (PFTE), le bisulfure de molybdène (MoS), le nitrure de bore (BN), l'huile de silicone ou également d'autres substances huileuses à cireuses.

8. Seringue (1) selon l'une des revendications 1 à 7, **caractérisée en ce que**,
à l'intérieur du piston de seringue (7), en partant de l'extrémité proximale (7a) (surface) jusqu'à l'extrémité distale (7b) (surface) du piston de seringue, au moins une lumière oblongue continue (14) s'étend à travers l'ensemble du piston de seringue (7),
dans lequel est disposé un piston intérieur (8) mobile ayant une extrémité proximale (8a) et une extrémité distale (8b), avec des ailettes (9) à l'extrémité distale.

9. Seringue (1) selon l'une des revendications 1 à 8, **caractérisée en ce qu'**une canule (2a) avec un connecteur (4b) ou avec une partie (4c) s'adaptant à la partie (4a) du système de connecteurs est disposée sur la sortie proximale de la seringue (11) sur le connecteur (4) ou sur la partie d'un système de connecteurs (4a).

10. Seringue (1) selon l'une des revendications 1 à 9, **caractérisée en ce que** le corps de seringue (7) avec le piston de seringue (7) mobile axialement inséré présente un volume de 0,01 ml à 2 ml ou le volume est de 0,2 ml à 1,5 ml.

11. Seringue (1) selon l'une des revendications 1 à 10, **caractérisée en ce que** le frottement (A) entre le piston de seringue (7) et le corps de seringue (5) est supérieur au frottement (B) entre le piston interne (8) dans la lumière (14) du piston de seringue (7), le frottement (A) étant supérieur de 10% au frottement (B) ou le frottement (A) étant supérieur de 50% au frottement (B).

12. Seringue (1) selon l'une des revendications 1 à 11, **caractérisée en ce que**
a) l'extrémité distale (7b) du piston de seringue (7) présente une aile (12) dans une matière thermoplastique, et/ou
b) l'extrémité proximale (7a) du piston de seringue (7) présente, proximalement et/ou dans la zone proximale sur le pourtour extérieur, une lèvre d'étanchéité ou un joint torique, la lèvre d'étanchéité ou le joint torique étant dans une matière thermoplastique, un élastomère ou un élastomère thermoplastique.

13. Seringue (1) selon l'une des revendications 1 à 12, **caractérisée en ce que**
a) l'aile (9) du piston intérieur (8) présente, à l'extrémité distale (8b), une came d'arrêt latérale qui est encliquetée dans un renfoncement dans l'aile (12) du piston de seringue (7) à l'état de stockage de la seringue à l'intérieur à l'extrémité distale dans la lumière (14), ou
b) à l'extrémité distale (7b), à l'intérieur de la lumière de l'aile (12) du piston de seringue (7), une came d'arrêt latérale qui est encliquetée dans un renfoncement dans l'aile (9) du piston intérieur (8) à l'état de stockage de la seringue à l'extérieur à l'extrémité distale (8b,) ou
c) le piston de seringue (7) est réalisé sous la forme d'au moins un piston qui présente, à l'intérieur de la lumière (14) à l'extrémité distale (7b), au moins une came d'arrêt latérale qui est encliquetée dans un renfoncement à l'état de stockage sur le côté extérieur du piston intérieur (8) à l'extrémité distale (8b), ou
d) le piston intérieur (8) est réalisé sous la forme d'au moins un piston qui présente, sur le côté extérieur, au moins une came d'arrêt qui est encliquetée dans au moins un renfoncement dans le piston de seringue (7) à l'état de stockage à l'intérieur de la lumière (14).

14. Kit comprenant une seringue (1) selon l'une des revendications 1 à 13, dans lequel la seringue est remplie d'une composition pharmaceutique ou dentaire liquide ou pâteuse, avec un scellant de fissures dentaire, une pâte de rétraction gingivale, un astringent, un adhésif dentaire, un adhésif automordançant, un adhésif tissulaire, avec un adhésif cutané cyanoacrylate, une solution de protéines adhésives, une solution de thrombine avec chlorure de calcium, un principe actif pharmaceutique, tel qu'un anesthésique ou des antibiotiques.
